# EUROPEAN PATENT APPLICATION

(11) **EP 2 332 923 A1**
(43) Date of publication of application: **15.06.2011**
(21) Application number: 09812905.9
(22) Date of filing: 11.09.2009
(51) Int. Cl.: C07D 239/47, C07D 401/12

(54) **5-Ý2-(METHYLTHIO)ETHOXY¨PYRIMIDINE-2-AMINE MANUFACTURING METHOD**

(30) Priority: 12.09.2008 JP 2008234299
(71) Applicant: Kowa Company, Ltd., Nagoya-shi, Aichi-ken 460-8625 (JP)
(72) Inventor: YAMAZAKI, Koichi, Higashimurayama-shi Tokyo 189-0022 (JP); KUSAKABE, Taichi, Higashimurayama-shi Tokyo 189-0022 (JP); OHGIYA, Tadaaki, Higashimurayama-shi Tokyo 189-0022 (JP); SHIBUYA, Kimiyuki, Higashimurayama-shi Tokyo 189-0022 (JP)
(74) Representative: Polypatent
(86) International application number: PCT/JP2009/004513
(87) International publication number: WO 2010/029756

(57) **Abstract**

A novel method for preparing 5-[2-(methylthio)ethoxylpyrimidin-2-amine useful as a regent or raw material for manufacture of medicaments, agricultural chemicals, and industrial products, which comprises alkanoylating the amino group of a 2-amino-4-alkoxypyrimidine, then converting the resultant into a 4-hydroxypyrimidine compound by a treatment with anhydrous aluminum chloride, further etherifying the 4-hydroxypyrimidine compound by a reaction with a 2-haloethyl methyl sulfide in the presence of a base, and removing the alkanoyl group to obtain 5-[2-(methylthio)ethoxylpyrimidin-2-amine.

## Description

### Technical Field

The present invention relates to a method for preparing 5-[2-(methylthio)ethoxy]pyrimidin-2-amine which is useful as a raw material for manufacturing medicaments, agricultural chemicals, and industrial products.

### Background Art

5-[2-(Methylthio)ethoxy]pyrimidin-2-amine is a compound useful as a reagent or raw material for manufacture of medicaments, agricultural chemicals, and industrial products. For example, the compound can be an important preparation raw material of 3-{1-[({3-[(cyclopentylmethyl)(ethyl)amino]-6-methoxypyridin-2-yl}methyl){5-[2-(methylsulfonyl)ethoxy]pyrimidin-2-yl}amino]methyl}-5-(trifluoromethyl)benzonitrile represented by the following formula (A), which is a pyrimidine compound with a novel dibenzylamine structure having a cholesterol ester transfer protein (CETP) inhibitory activity, and is useful as a prophylactic and/or therapeutic agent for hyperlipidemia, arteriosclerosis, heart diseases, and the like (Patent document 1).

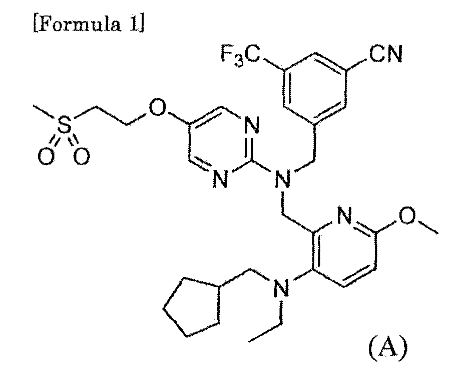

As a method for preparing a 5-[2-(methylthio)ethoxy]pyrimidin-2-amine derivative, method 1) is known which comprises the step of a substituting reaction of the chlorine atom of available 5-bromo-2-chloropyrimidine used as a starting material with an amine compound, which forms an important constituent moiety of the target compound, successive steps of replacement of the bromine atom with higher reactive iodine atom and then reaction with benzyl alcohol as an unit for introducing hydroxyl group, followed by deprotection and etherification to prepare a 6-alkaxypyrimidine derivative (Patent document 2). This reaction scheme is shown below with reaction formulas.

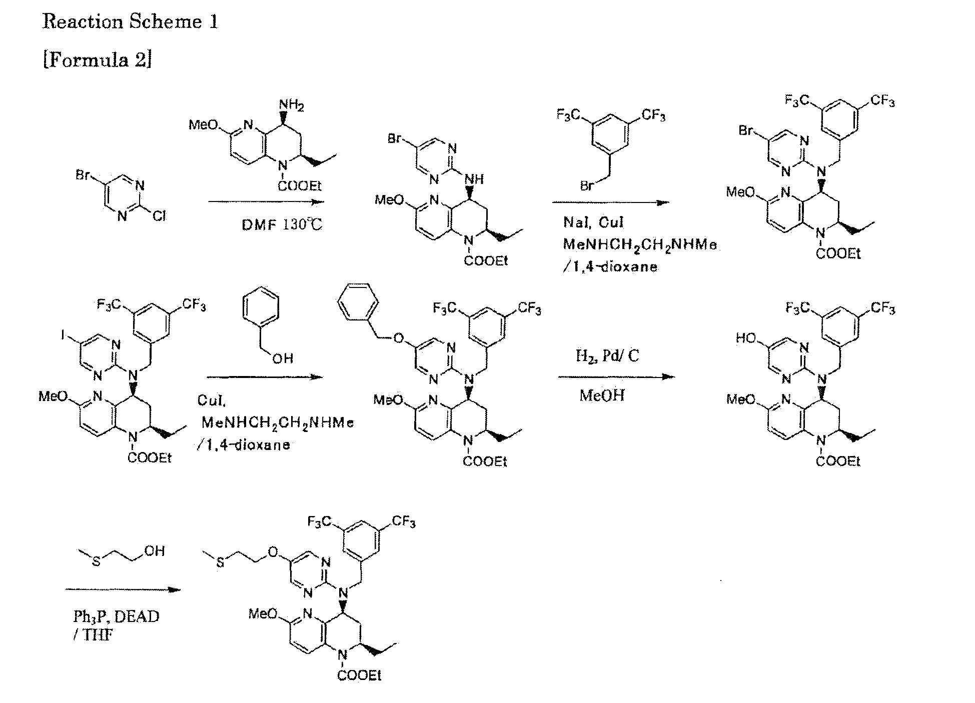

Another method 2) is known which comprises the step of preparation of 2-chloro-5-(4,4,5,5-tetramethyl-[1,3,2]dioxaboron-2-yl)pyrimidine having boronic acid ester at the 5-position, that is easily converted into hydroxyl group, according to a known method by using 5-broma-2-chloropyrimidine as a starting material, successive step of substitution reaction of the chlorine atom with an amine compound, followed by the step of conversion of the boron substituent to hydroxyl group by a treatment with hydrogen peroxide to afford the desired target compound (Patent document 3). This reaction scheme is shown below with reaction formulas.

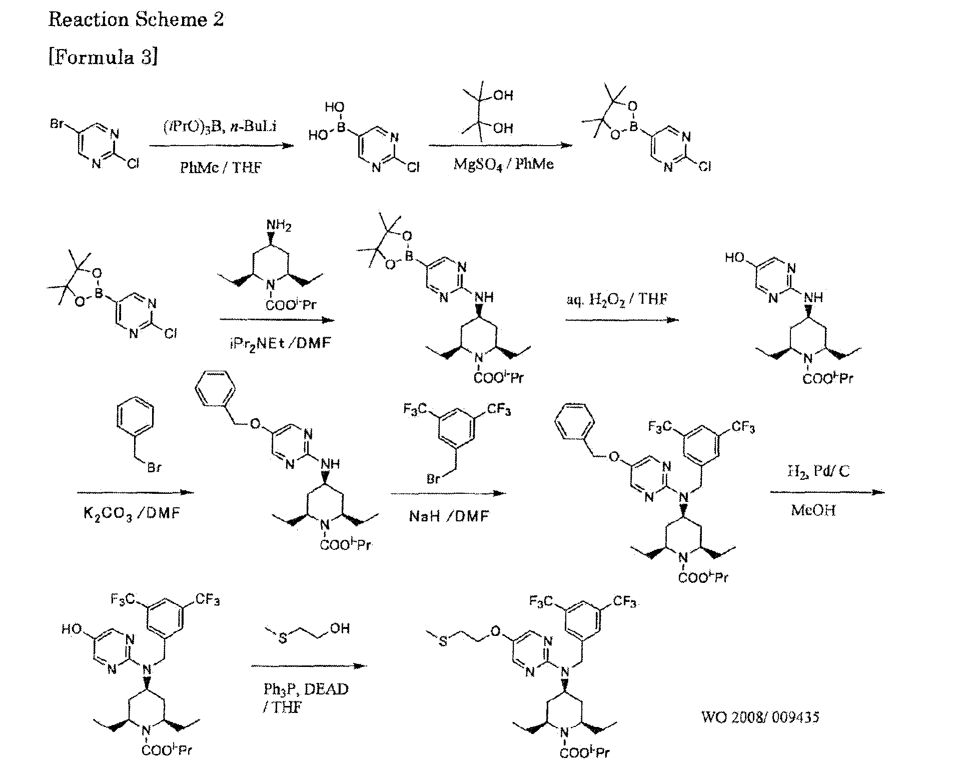

Since it is difficult to construct the pyrimidine ring moiety in a later stage, which is a partial structure important for expression of the desired physiological activity, these methods comprise steps of first incorporating the pyrimidine ring moiety into a complicated compound structure, and then repeating stepwise substitution reaction and derivatization. However, these methods are serially performed synthetic methods, and accordingly, they have a problem that a total yield decreases with accumulative progress of the reaction steps for preparation of the desired compound. Therefore, these preparation methods are extremely inefficient for a purpose of manufacturing the target compound in a large scale.

A method for obtaining the desired 5-[2-(methylthio)ethoxy]pyrimidin-2-amine is also reported, which comprises the step of substitution reaction of the chlorine atom of 5-bromo-2-chloropyrimidine used as a starting material with 4-methoxybenzylamine to prepare a 2-aminopyrimidine compound, successive step of substitution reaction of the bromine atom with 2-methylthioethanol in the presence of copper iodide, followed by debenzylation with an acid. This method constitutes a part of a parallel synthetic method which is different from the aforementioned serially performed synthetic methods, and the steps are also shortened to three steps. However, this method has a problem that separation of by-products and purification are complicated, and the total yield is also low (18%). The method also has a problem that the starting material, 2-chloro-5-bromapyrimidine, is very expensive, and therefore the method is not suitable as a large-scale manufacturing process (Patent document 1). The reaction Scheme is shown below with reaction formulas.

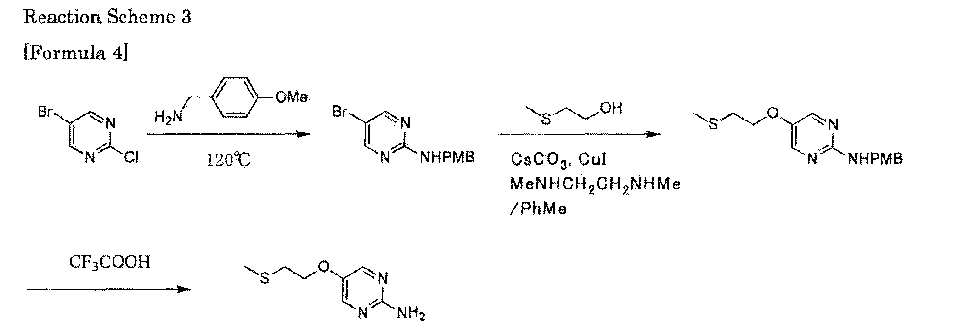

### Prior Art References

### Patent documents

Patent document 1: International Patent Publication WO2008/018529
Patent document 2: International Patent Publication WO2005/095396
Patent document 3: International Patent Publication WO2008/0094

### Summary of the Invention

### Object to be Achieved by the Invention

An object of the present invention is to provide a method for efficiently preparing 5-[2-(methylthio)ethoxy]pyrimidin-2-amine. More specifically, the object of the present invention is to provide a method for obtaining the aforementioned compound in a high yield with high purity under a mild reaction condition.

### Means for Achieving the Object

The inventors of the present invention conducted various researches to achieve the aforementioned object. As a result, they found that, as shown in the following reaction formulas, a 5-hydroxypyrimidine compound was successfully obtained in a high yield by using more inexpensive aluminum chloride in the dealkylation reaction of a 2-alkanoylamino-5-alkoxypyrimidine (see, Comparative Example 1 which will be mentioned later), and that the target 5-[2-(methylthio)ethoxy]pyrimidin-2-amine was successfully prepared in a high yield by using a 2-haloethyl methyl sulfide, being an inexpensive and stable reagent, to allow reaction under a basic condition in the successive etherification reaction, followed by hydrolyzation with a base.

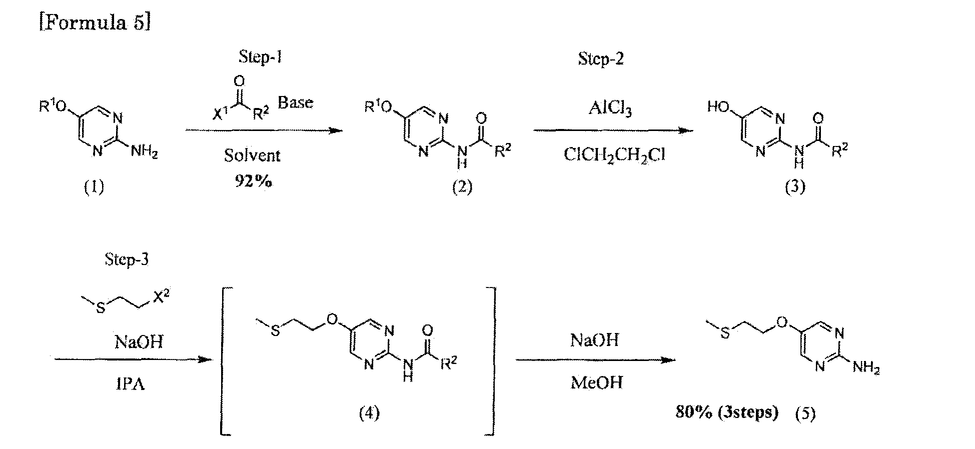

### [In the formulas, R¹ and R² represent a lower alkyl group, and X¹ and X² represent a halogen atom.]

The present invention thus provides the preparation methods according to the following [1] to [14].
[1] A method for preparing 5-[2-(methylthio)ethoxy]pyrimidin-2-amine represented by the following formula (5): 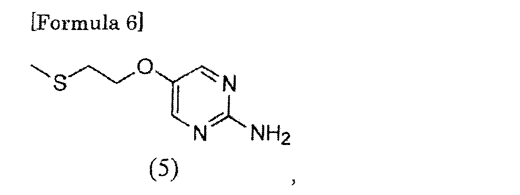 which comprises the following steps:
   a) the step of reacting a compound represented by the following formula (3): 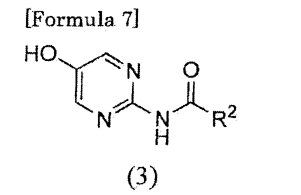 [in the formula, R² represents a lower alkyl group] with a 2-haloethyl methyl sulfide in an alcoholic solvent in the presence of an alkaline metal hydroxide to prepare a compound represented by the following formula (4): 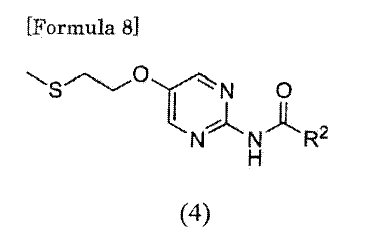 [in the formula, R² represents the same group as defined above], and
   b) the step of treating the compound represented by the formula (4) with a base in a solvent to prepare a compound represented by the formula (5);

[2] The method according to [1] mentioned above, wherein the 2-haloethyl methyl sulfide is 2-chloroethyl methyl sulfide;
[3] The method according to [1] or [2] mentioned above, wherein R² is n-pentyl group;
[4] The method according to any one of [1] to [3] mentioned above, wherein the alkaline metal hydroxide is sodium hydroxide;
[5] The method according to any one of [1] to [4] mentioned above, wherein the alcoholic solvent is methanol, ethanol, isopropanol, t-butanol, or an arbitrary mixture of these
[6] The method according to any one of [1] to [5] mentioned above, wherein the step b) is successively performed without isolating the compound of the formula (4) obtained in the step a);

[7] The method according to any one of [1] to [6] mentioned above, which further comprises the step of treating a compound represented by the following formula (2): 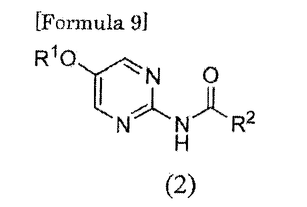 [in the formula, R¹ and R² independently represent a lower alkyl group] with aluminum chloride in a halogenated hydrocarbon solvent to prepare a compound represented by the formula (3);
[8] The method according to [7] mentioned above, wherein R¹ is methyl group;
[9] The method according to [7] or [8] mentioned above, wherein the halogenated hydrocarbon solvent is 1,2-dichloroethane;

[10] The method according to any one of [7] to [9] mentioned above, which further comprises the step of reacting a compound represented by the following formula (1): 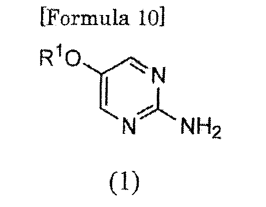 [in the formula, R¹ represents a lower alkyl group] with an alkanoic acid halide in a solvent in the presence of an organic base to prepare a compound represented by the formula (2);
[11] The method according to [10] mentioned above, wherein the organic base is pyridine;
[12] The method according to [10] or [11] mentioned above, wherein the alkanoic acid halide is hexanoyl chloride;
[13] The method according to any one of [10] to [12] mentioned above, wherein the solvent is a halogenated hydrocarbon solvent; and
[14] A method for preparing 3-{1-[({3-[(cyclopentylmethyl)(ethyl)amino]-6-methoxypyridin-2-yl}methyl){5-[2-(methylsulfonyl)ethoxy]pyrimidin-2-yl}amino]methyl}-5-(trifluoroulethyl)benzonitrile, which comprises the steps described in any one of [1] to [13] mentioned above.

### Effect of the Invention

According to the method of the present invention, 5-[2-(methylthio)ethoxylpyrimidin-2-amine useful as a raw material for manufacture of medicaments, agricultural chemicals, and industrial products can be efficiently prepared under a convenient reaction condition. By utilizing the method of the present invention, a 5-[2-(methylthio)ethoxy]pyrimidin-2-amine derivative, including for example 3-{1-[({3-[(cyclopentylmethyl)(ethyl)amino]-6-methoxypyridin-2-yl}methyl){5-[2-(methylsulfonyl)ethoxy]pyrimidin-2-yl}amino]methyl}-5-(trifluoromethyl)benzonitrile, can be prepared, and a highly pure target compound can be efficiently prepared in a high yield by avoiding reactions such as the Mitsunobu reaction that requires precise setting of reaction conditions as well as complicated reaction treatment and purification process, and also avoiding decrease in production yield due to multiple reaction steps, purification in each step, and the like.

### Modes for Carrying out the Invention

As the lower alkyl group as R¹ and R², for example, a linear or branched alkyl group having 1 to 6 carbon atoms can be used, and examples include, for example, methyl group, ethyl group, n-propyl group, isopropyl group, n-butyl group, s-butyl group, t-butyl group, n-pentyl group, n-hexyl group, and the like.
Examples of the halogen atom as X¹ and X² include chlorine atom, bromine atom, and iodine atom.

Hereafter, each reaction step of the preparation method of the present invention will be explained.

### 1. Step for preparing 2-alkanoylamino-5-alkoxypyrimidine (2)

Type of the acid halide used for this reaction is not particularly limited. Examples include, for example, acetyl chloride, propanoyl chloride, butanoyl chloride, trimethylacetyl chloride, cyclohexylcarbonyl chloride, pentanoyl chloride, hexanoyl chloride, trichloroacetyl chloride, trifluoroacetyl chloride, and the like, and more preferred examples include hexanoyl chloride. Preparation of 5-[2-(methylthio)ethoxy]pyrimidin-2-amine can be achieved by applying a method wherein a 2-acylamino-5-alkoxypyrimidine produced from an aromatic acyl halide such as benzoyl chloride, p-fluorobenzoyl chloride and p-nitrabenzoyl chloride is used as an intermediate. However, a 5-hydroxypyrimidine compound obtained by dealkylation easily migrates in an aqueous layer at the time of extraction operation. Accordingly, it is desirable from an industrial viewpoint to choose an acid halide that is relatively inexpensive among highly lipophilic alkanoic acid halides and allows rapid deacylation as the subsequent deprotection.

Although amount of the acid halide used is not particularly limited, the acid halide is preferably used in an amount of, for example, about 1 to 1.5 moles, more preferably about 1.1 moles, with 1 mole of 5-alkoxypryimidin-2-amine (1).
The base used for the reaction with the aforementioned acid halide is not particularly limited. Examples include, for example, organic bases such as pyridine, lutidine, collidine, N,N-dimethylaminopyridine, DBU, DBN, DABCO, triethylamine, N,N-diisopropylethylamine, N,N-diisoprapylpentylamine, and trimethylamine. It is more preferable to use pyridine.

This reaction can be performed in the presence or absence of a solvent. The reaction is preferably performed in a solvent. A solvent to be used is not particularly limited so long as the solvent is inert in the reaction. Examples include, for example, ether type solvents such as diethyl ether, tetrahydrofuran, 1,4-dioxane, 1,3-dioxane, t-butyl methyl ether, monoglyme and diglyme, aromatic hydrocarbon solvents such as benzene, toluene, xylene, mesitylene, chlorobenzene and 1,2-dichlorobenzene, aliphatic hydrocarbon solvents such as n-hexane, cyclohexane, n-octane and n-decane, halogenated hydrocarbon solvents such as methylene chloride, dichloroethane, chloroform and carbon tetrachloride, water, and the like. Among them, ether type solvents such as diethyl ether, tetrahydrofuran, 1,4-dioxane, 1,3-dioxane, t-butyl methyl ether, monoglyme and diglyme, halogenated hydrocarbon solvents such as methylene chloride, dichloroethane, chloroform and carbon tetrachloride, and water are preferred, and methylene chloride and chloroform are particularly preferred. These solvents may be used alone, or arbitrary two or more kinds of solvents can also be used in combination. Amount of the solvent used is not particularly limited.
The reaction may be ordinarily performed at a reaction temperature in the range of 80 to 50°C, preferably 20 to 30°C. Reaction time is usually preferred to be 5 minutes to 12 hours, more preferably 10 minutes to 10 hours.

### 2. Step of preparing 5-hydroxypyrimidine compound (3)

This step is a step of subjecting the 5₋alkoxypyrimidine compound (2) to a dealkylation to prepare the 5-hydroxypyrimidine compound (3).
Aluminum chloride used for this reaction is anhydrous aluminum chloride. The reagent is used in an amount of, for example, 1 to 5 moles with 1 mole of the 5-alkoxypyrimidine compound (2), and can be preferably used in an amount of 3 moles with 1 mole of the 5-alkoxypyrimidine compound (2).

Although this reaction can be performed in the presence or absence of a solvent, the reaction is preferably performed in a solvent. A solvent to be used is not particularly limited so long as the solvent is inert in the reaction. Examples include, for example, aromatic hydrocarbon solvents such as benzene, toluene, xylene, mesitylene, chlorobenzene, 1,2-dichlorobenzene and nitrobenzene, aliphatic hydrocarbon solvents such as n-hexane, cyclohexane, n-octane and n-decane, halogenated hydrocarbon solvents such as methylene chloride, 1,2-dichloroethane, chloroform and carbon tetrachloride, and the like. Among them, halogenated hydrocarbon solvents such as methylene chloride, dichloroethane, chloroform and carbon tetrachloride are preferred, and chloroform and dichloroethane are particularly preferred. These solvents may be used alone, or arbitrary two or more kinds of solvents can also be used in combination. Amount of the solvent used is not particularly limited.

The reaction may be usually performed at a reaction temperature in the range of 0 to 180°C, preferably 50 to 90°C. Reaction time is usually preferred to be 0.5 to 15 hours, more preferably 0.5 to 3 hours.
After completion of the reaction, the reaction mixture can be poured into ice water, and the target compound can be extracted with an organic solvent such as methylene chloride, chloroform, and ethyl acetate. It is preferable to add aqueous sodium hydroxide and methanol to the reaction mixture under ice cooling to convert unreacted aluminum chloride and aluminum complex into aluminum hydroxide, further add chloroform to sufficiently dissolve the target compound, then remove insoluble solids by Celite filtration, and extract the target compound with a mixture of chloroform and methanol.

The dealkylation of the aromatic alkyl ether can be performed by referring to a method generally used as deprotection conditions for the protective group (Protective Groups in Organic Synthesis Fourth Edition, John Wiley & Sons, Inc.). In this reaction, the dealkylated 5-hydroxypyrimidine compound (3) easily moves to the aqueous layer when an ordinary extraction operation is performed, which results in low extraction efficiency in the organic layer and lowered recovery ratio. However, this problem can be avoided by using a highly lipophilic alkanoic acid halide such as hexanoyl chloride in the precedent step as described above.

If a Lewis acid such as iodotrimethylsilane, borane tribromide, borane trichloride and borane triiodide is used in the dealkylation of the aromatic alkyl ether, the reaction advances quickly. However, it generally becomes necessary to separate by-products formed during the reaction by silica gel chromatography or the like. As the dealkylation reaction, a reaction in an acidic solution such as those of 48% hydrogen bromide and acetic acid or hydroiodic acid, and a reaction using a nucleophilic agent such as lithium ethanethiolate, sodium ethanethiolate, and lithium iodide are known. However, these reactions may result in a low yield or may not advance, or may give complex mixture resulted from hydrolysis of alkanoic acid amide accompanying the demethylation. Therefore, it is not preferable to use these reactions in this step. In contrast, according to the method of the present invention, the compound (3) can be obtained in an extremely high yield by a convenient post-treatment after the reaction under a mild reaction condition.

### 3. Step of preparing 5-[2-(methylthio)ethoxy]pyrimidin-2-amine (5)

This step is to subject the 5-hydroxypyrimidine compound (3) to an etherification with a 2-halaethyl methyl sulfide in the presence of a base to prepare 5-[2-(methylthio)ethoxy]pyrimidin-2-amine (5).
As the 2-haloethyl methyl sulfide used for this reaction, 2-chloroethyl methyl sulfide is preferred, and the sulfide can be used in an amount of, for example, 1 to 4 moles, preferably 1 to 2 moles, with 1 mole of the 5-hydroxypyrimidine compound (3).

The base used for this reaction is not particularly limited. For example, alkaline metal hydrides such as lithium hydride, sodium hydride and potassium hydride, simple substances of alkali metal such as metallic lithium, metallic sodium and metallic potassium, alkaline metal hydroxides such as lithium hydroxide, sodium hydroxide, potassium hydroxide and cesium hydroxide, alkali metal carbonates such as lithium carbonate, sodium carbonate, potassium carbonate and cesium carbonate, lithium diisopropylamide, sodium diisopropylamide, potassium diisopropylamide, Lithium hexamethyldisilazide, sodium hexamethyldisilazide, potassium laexamethyldisilazide, sodium t-butoxide, potassium t-butoxide, n-butyllithium, s butyllithium, t-butyllithium, and the like can be used. The base can be used in an amount of, for example, 1 to 5 moles, preferably 1 to 2 moles, with 1 mole of the 5-hydroxypyrimidine compound (3).

Although this reaction can be performed in the presence or absence of a solvent, the reaction is preferably performed in a solvent. A solvent to be used is not particularly limited so long as the solvent is inert in the reaction. Examples include, for example, alcoholic solvents such as methanol, ethanol, n-prapanol, isopropanol, n butanol, s-butanol and t-butanol, and isopropanol and t-butanol are preferred. Acetonitrile, tetrahydrofuran, dimethyl sulfoxide, N,N-dimethylformamide, N-methylpyrrolidone, water and the like can also be used. These solvents may be used alone, or arbitrary two or more kinds of solvents can also be used in combination. Amount of the solvent used is not particularly limited.
The reaction may be usually performed at a reaction temperature in the range of 0 to 120°C, preferably 70 to 100°C. Reaction time is usually preferred to be 1 to 24 hours, more preferably 3 to 11 hours.

After the 2-alkanoylamino-5-[2-(methylthio)ethoxy]pyrimidine obtained by the etherification is subjected to a post-treatment, the desired 5-[2-(methylthio)ethoxylpyrimidin-2-amine (5) may be isolated, or methanol and 2 M aqueous sodium hydroxide may be successively added to the reaction solution without such isolation, and the mixture may be used for the hydrolysis of the amide compound. The solvent used for this reaction may be, besides methanol, an alcoholic solvent such as ethanol, propanol, isopropanol, and butanol. After completion of the reaction, the target compound can be isolated by, for example, performing an ordinary extraction operation, and then performing a purification method ordinarily used in the synthetic organic chemistry such as recrystallization, various kinds of chromatography, and distillation as required.

### Examples

The present invention will be more specifically explained with reference to examples. However, the scope of the present invention is not limited to the following example.

### Example 1: Preparation of N-(5-methoxypyrimidin-2-yl)hexanamide

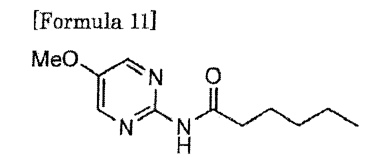

A solution of 2-amino-5-methoxypyrimidine (83.4 g, 667 mmol) in dichloromethane (800 mL) was added with pyridine (132 g, 1.67 mol), the mixture was added with hexanoyl chloride (98.7 g, 733 mmol) under ice cooling, and the mixture was stirred for 30 minutes at room temperature. The reaction mixture was added with 1 M aqueous glycine (670 mL) under ice cooling, and the mixture was stirred for 1 hour, and then extracted with chloroform (300 mL x 3). The organic layer was washed successively with aqueous NaHCO₃ and saturated brine, dried over anhydrous sodium sulfate, and concentrated under reduced pressure, and the resulting residue was azeotroped with toluene The resulting residue was recrystallized from chloroform/hexane to obtain pale yellow solid (137 g, 92%).
Melting point: 101. 7-102.2°C
IR (ATR) cm⁻¹_{:} 2951, 1666, 1581, 1514, 1435, 1390, 1274
¹H-NMR (400 MHz, CDCl₃) δ : 0.91 (3H, t, J = 7.1 Hz), 1.30-1.40 (4H, m), 1.70-1.78 (2H, m), 2.50-2.70 (2H, m), 3.89 (3H, s), 8.10 (1H, br), 8.28 (2H, s)
Elemental analysis: as C₁₁H₁₇N₃O₂
Calculated : C, 59.17; H, 7.67; N, 18.82
Found: C, 59.12; H, 7.66; N, 18.92

### Example 2: Preparation of N-(5-hydroxypyrimidin-2-yl)hexanamide

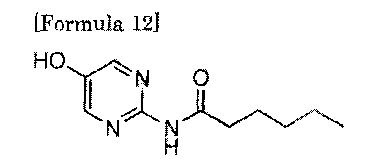

N-(5-Methoxypyrimidin-2-yl)hexanamide (5.0 g, 22.4 mmol) was dissolved in 1,2-dichloroethane (50 mL), the solution was added with aluminum chloride (9.0 g, 67.2 mmol) under ice cooling, and the mixture was stirred at 70°C for 2 hours under an argon atmosphere. The reaction mixture was added with 2 M NaOH (34 mL) and methanol (20 mL) with vigorous stirring under ice cooling, and the mixture was stirred at room temperature for 1 hour, and then added with chloroform (150 mL)). The reaction mixture was filtered through Celite, and washed with chloroform : methanol (10:1), the filtrate was added with saturated brine (50 mL) and extracted with chloroform:methanol (10:1, 250 mL x 2), and the organic layer was dried over anhydrous sodium sulfate, and then concentrated under reduced pressure to obtain pale brown solid (crude product, 4.70 g, 100%).
Melting point: 141.8-142.8°C
IR (ATR) cm⁻¹: 2951, 2931, 2858, 1629, 1586, 1514, 1444
¹H-NMR(400MHz, d₆-DMSO) δ: 0.86 (3H, t, J = 7.3 Hz), 1.24-1.31 (4H, m), 1.51-1.58 (2H, m), 2.35 (2H, t, J = 7.3 Hz), 8.20 (2H, s), 10.09 (1H, br s), 10.2 1(1H, s)
Elemental analysis: as C₁₀H₁₅N₃O₂
Calculated: C, 57.40; H, 7.23; N, 20.08
Found: C, 57.45; H, 7.28; N, 20.06

### Example 3: Preparation of 5-[2-(methylthio)ethoxy]pyrimidin-2-amine

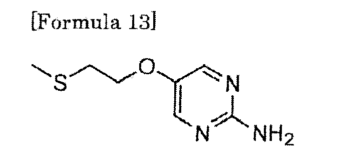

A suspension of N-(5-hydroxypyrimidin-2-yl)hexanamide (5.0 g, 23.9 mmol) in isopropanol (50 mL) was added successively with sodium hydroxide (956 mg, 23.9 mmol) and 2-chloroethyl methyl sulfide (2.65 g, 23.9 mmol), and the mixture was stirred at 90°C for 3 hours. The reaction mixture was added successively with methanol (50 mL) and 2 M NaOH (50 mL), and the mixture was stirred at 70°C for 1 hour. The reaction mixture was added with water (150 mL), and extracted with chloroform (150 mL x 2). The organic layer was washed with 1 M aqueous HCl (150 mL x 2), the aqueous layers were combined, made alkaline with 2 M aqueous NaOH, and extracted with chloroform (150 mL x 2), and the organic layers were combined, dried over anhydrous sodium sulfate, and then concentrated under reduced pressure to obtain pale yellow solid (3.53 g, 80% for two steps).
Melting point: 85.6-87.6°C
IR (ATR) cm⁻¹: 3473, 3299, 3171, 2918, 1635, 1564, 1471, 1456, 1270
¹H-NMR (400 MHz, CDCl₃) δ : 2.21 (3H, s), 2.85 (2H, t, J = 6.6 Hz), 4.13 (2H, t, J = 6.6 Hz), 4.93 (2H, br), 8.06 (2H, s).
Elemental analysis: as C7H₁₁N₃O₁S₁
Calculated: C, 45.39; H, 5.99; N, 22.68
Found: C, 45.33; H, 5.94; N, 22.72

### Example 4: Preparation of 5-[2-(methylthio)ethoxy]pyrimidin-2-amine

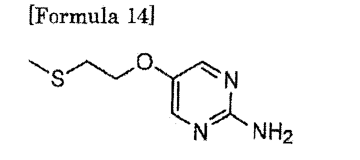

A suspension of N-(5-hydroxypyrimidin-2-yl)hexanamide (96.6 g, 0.462 mol) in isopropanol (1000 mL) was added successively with sodium hydroxide (18.5 g, 0.462 mol) and 2-chloroethyl methyl sulfide (51.1. g, 0.462 mol), and the mixture was stirred at 90°C for 3 hours. The reaction mixture was added successively with methanol (350 mL) and 2 M NaOH (350 mL), and the mixture was stirred at 70°C for 1 hour. The reaction mixture was concentrated under reduced pressure, then added with water (150 mL), and extracted with chloroform (1000 mL). The organic layer was washed with 1 M aqueous HCl (500 mL x 2), the aqueous layers were combined, made alkaline with 2 M aqueous NaOH, and extracted with chloroform (750 mL x 2). The organic layers were combined, dried over anhydrous sodium sulfate, and then concentrated under reduced pressure to obtain pale yellow solid (66.6 g, 78% for two steps). ¹H-NMR (400MHz, CDCl₃) δ 2.21 (3H, s), 2.85 (2H, t, J = 6.6 Hz), 4.13 (2H, t, J = 6.6 Hz), 4.93 (2H, br), 8.06 (2H, s)

### Example 5: Preparation of 3-{1-[({3-[(cyclopentylmethyl)(ethyl)amino]-6-methoxypyridin-2-yl}methyl){5-[2-(methylsulfonyl)ethoxy]pyrimidin-2-yl}amino]methyl}-5-(trifluoromethyl)benzonitrile (A)

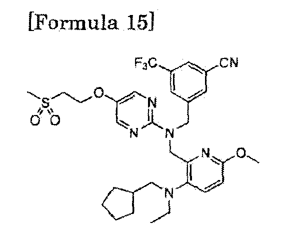

### Step 1: Synthesis of 3-[(cyclopentylmethyl)(ethyl)amino]-6-methoxypicolinaldehyde

A solution of (3-bromo-6-methoxypyridin-2-yl)methanol (10.6 g, 48.6 mmol)) synthesized by the method described in Organic & Bimolecular Chemistry, 1 (16), 2865-2876 (2003) in dichloromethane (150 mL) was successively added dropwise with N,N-diisoprapylethylamine (31.4 g, 243 mmol) and chloromethyl methyl ether (13.3 g, 165 mmol) under ice cooling. The mixture was warmed to room temperature, stirred for 16 hours, and then added with methanol (30 mL), and the mixture was stirred for 30 minutes. The reaction mixture was concentrated under reduced pressure, and the resulting residue was purified by using silica gel column chromatography (hexane:ethyl acetate = 30:1 → 20:1) to obtain 3-bromo-6-methoxy-2-[(methoxymethoxy)methyl]pyridine (12.1g, 95%) as pale yellow oil.
¹H-NMR (400 MHz, CDCl₃) δ: 3.46 (3H, s), 3.93 (3H, s), 4.73 (2H, s), 4.81 (2H, s), 6.59 (1H, d, J = 8.7 Hz), 7.68 (1H, d, J = 8.7 Hz)

A solution of 3-bromo-6-methoxy-2-[(methoxymethoxy)methyl]pyridine (1.50 g, 5.72 mmol), tris(dibenzylideneacetane)(chloroform)dipalladium(0) (592 mg, 0.572 mmol), (2-biphenyl)di-t-butylphosphine (680 mg, 2.28 mmol), sodium t-butoxide (1.65 g, 17.2 mmol), and ethylamine (2.0 M tetrahydrofuran solution, 15 mL, 30 mmol)) in tetrahydrofuran (5 mL) was heated to 135°5 over 3 minutes by microwave irradiation (500 W). The reaction mixture was cooled, then filtered through Celite, and washed with ethyl acetate. The reaction mixture was concentrated under reduced pressure, and the resulting residue was purified by using silica gel column chromatography (hexane:ethyl acetate = 8:1) to obtain N-ethyl-6-methoxy-2-[(methoxymethoxy)methyl]pyridin-3-amine (954 mg, 74%) as yellow oil.
¹H-mm (400 MHz, CDCl₃) δ: 1.27 (3H, t, J = 7.1 Hz), 3.12 (2H, q, J = 7.1 Hz), 3.43 (3H, s), 3.86 (3H, s), 4.23 (1H br s), 4.68 (2H, s), 4.71 (2H, s), 6.64 (1H, d, J = 8.8 Hz), 7.03 (1H, d, J = 8.8 Hz)

A solution of N-ethyl -6 -methoxy-2-[(methoxymethoxy)methyl]pyridin-3-amine (7.20 g, 31.8 mmol) and cyclopentane carbaldehyde (3.75 g, 38.2 mmol) in 1,2-dichloroethane (240 mL) was added with sodium triacetoxyborohydride (88.75 g, 41.3 mmol), and the mixture was stirred at room temperature for 12 hours. The reaction mixture was added with water, and extracted with chloroform. The organic layers were combined, washed with water and saturated brine, dried over anhydrous sodium sulfate; and concentrated under reduced pressure. The resulting residue was purified by using silica gel column chromatography (hexane:ethyl acetate = 8:1) to obtain N-(cyclopentylmethyl)-N-ethyl-6-methoxy-2-[(methoxymethoxy)methyl]pyridin-3-amine (8.39 g, 86%) as pale yellow oil.
¹H-NMR (400 MHz, CDCl₃) δ : 0.94 (3H, t, J = 7.1 Hz), 1.05-1.23 (2H, m), 1.34-1.70 (6H, m), 1.82 (1H, m), 2.78 (2H, d, J = 7.5 Hz), 2.90 (2H, q, J = 7.1 Hz), 3.47 (3H, s), 3.93 (3H, s), 4.76 (2H, s), 4.85 (2H, s), 6.67 (1H, d, J = 8.8 Hz), 7.46 (1H, d, J = 8.8 Hz)

A solution of N-(cyclopentylmethyl)-N-ethyl-6-methoxy-2-[(methoxymethoxy)methyl]pyridin-3-amine (8.39 g, 27.2 mmol) in a mixture of dioxane (400 mL) and water (100 mL) was added dropwise with concentrated hydrochloric acid (20 mL), and the mixture was stirred at 50°C for 19 hours. The reaction mixture was made basic with aqueous sodium hydroxide, and extracted with ethyl acetate. The organic layers were combined, washed with water and saturated brine, dried over anhydrous sodium sulfate, and concentrated under reduced pressure. The resulting residue was purified by using silica gel column chromatography (hexane:ethyl acetate = 15:1) to obtain {3-[(cyclopentylmethyl)(ethyl)amino]-6-methoxypyridin-2-yl}methanol (6.72 g, 94%) as pale yellow oil.
¹H-NMR (400 MHz, CDCl₃) δ : 0.96 (3H, t, J = 7.1 Hz), 1.05-1.23 (2H, m), 1.33-1.77 (6H, m), 1..87 (1H, m), 2.77 (2H, d, J = 7.5 Hz), 2.86 (2H, q, J = 7.1 Hz), 3.94 (3H, s), 4.79 (2H, s), 5.04 (1H, br s), 6.64 (1H, d, J = 8.7 Hz), 7.49 (1H, d, J = 8.7 Hz)

A solution of {3-[(cyclopentylmethyl)(ethyl)amino]-6-methoxypyridin-2-yl}methanol (2.0 g, 7.57 mmol) in chloroform (200 mL) was added with manganese dioxide (20 g, 230 mmol), and the mixture was stirred at 55°C for 16 hours. The reaction mixture was filtered through Celite, and the filtrate was washed with chloroform, and then concentrated under reduced pressure. The resulting residue was added with chloroform (200 mL) and manganese dioxide (20 g, 230 mmol), and the mixture was stirred at 55°C for 6 hours. The reaction mixture was filtered through Celite, and the filtrate was washed with chloroform, and then concentrated under reduced pressure to obtain 3-[(cyclopentylmethyl)(ethyl)amino]-6-methoxypicolinaldehyde (1.68 g, 85%) as pale yellow oil.
¹H-NMR (400 MHz, CDCl₃) δ : 1.02 (3H, t, J = 7.1 Hz), 1..05-1.23 (2H, m), 1.36-1.73 (6H, m), 1.95 (1H, m), 2.99 (2H, d, J = 7.6 Hz), 3.13 (2H, q, J = 7.1 Hz), 3.99 (3H, s), 6.93 (1H, d, J = 9.0 Hz), 7.59 (1H, d, J = 9.0 Hz), 10.4 (1H, s)

### Step 2: Preparation of 3-{1-[({3-[(cyclopentylmethyl)(ethy)amino]-6-methoxypyridin-2-yl}methyl){5-[2-(methylsulfonyl)ethoxy]pyrimidin-2-yl}amino]methyl}-5-(trifluoromethyl)benzonitrile

A solution of 5-[2-(methylthio)ethoxy]pyrimidin-2-amine (1.03 g, 5.55 mmol) and 3-[(cyclopentylmethyl)(ethyl)amino]-6-methoxypicolinaldehyde obtained in Step 1 (1.60 g, 6.10 mmol) in 1,2-dichloroethane (60 mL) was stirred at room temperature for 10 minutes, and then added with sodium triacetoxyborohydride (1.24 g, 5.83 mmol), and the mixture was stirred at room temperature for 12 hours. The reaction mixture was added with water, and extracted with chloroform. The organic layers were combined, washed with water and saturated brine, dried over anhydrous sodium sulfate, and concentrated under reduced pressure. The resulting residue was purified by using silica gel column chromatography (hexane:ethyl acetate = 5:1) to obtain N-({3-[(eyclopentylmethyl)(ethyl)amino]-6-methoxypyridin-2-yl}methyl)-5-[2-(methylthio)ethoxy]pyrimidin-2-amine (1.60 g, 67%) as pale yellow oil.
¹H-NMR (400 MHz, CDCl₃) δ : 0.97 (3H, t, J = 7.1 Hz), 1.08-1.25 (2H, m), 1.34-1.70 (6H, m), 1.84 (1H, m), 2.21 (3H, s), 2.81 (2H, d, J = 7.5 Hz), 2.85 (2H, t, J = 6.7 Hz), 2.91 (2H, q, J = 7.1 Hz), 3.94 (3H, s), 4.12 (2H, t, J = 6.7 Hz), 4.70 (2H, d, J = 4.6 Hz), 6.33 (1H, t, J = 4.6 Hz), 6.64 (1.H, d, J = 8.6 Hz), 7.47 (1H, d, J = 8.6 Hz), 8.12 (2H, s)

A solution of N-({3-[(cyclopentylmethyl) (ethyl)amino]-6-methoxypyridin-2-yl}methyl)-5-12-(methylthio)ethoxy]pyrimidin-2-amine (2.50 g, 5.79 mmol) in N,N-dimethylformamide (50 mL) stirred under ice cooling was added with sodium hydride (50% in oil, 1.11 g, 23.2 mmol), and the mixture was stirred at 50°C for 30 minutes. The reaction mixture was added with N,N-dimethylformamide (50 mL), the mixture was cooled to -78°C, and added with a solution of 3-bromomethyl-5-trifluoramethylbenzonitrile (3.06 g, 11.6 mmol) synthesized by the method described in Japanese Patent Unexamined Publication (Kokai) No. 2003-221376 in N,N-dimethylformamide (50 mL), and the mixture was stirred for 12 hours with warming to room temperature. The reaction mixture was added with water, the mixture was extracted with ethyl acetate, and the organic layer was washed with saturated brine, dried over anhydrous sodium sulfate, and concentrated under reduced pressure. The resulting residue was purified by using silica gel column chromatography (hexane:ethyl acetate = 20:1 → 10:1) to obtain the target compound, 3-{1-[({3-[(cyclopentylmethyl)(ethyl)amino]-6-methoxypyridin-2-yl}methyl){5-[2-(methylthio)ethoxylpyrimidin-2-yl}amino]methyl}-5-(trifluoromethyl)benzonitrile (2.77 g, 78%) as pale red oil.
¹H-NMR (400 MHz, CDCCl₃) δ 0.94 (3H, t, J = 7.0 Hz), 1.06-1.22 (2H, m), 1.35-1.70 (6H, m), 1.87 (1H, m), 2.20 (3H, s), 2.77 (2H, d, J = 7.4 Hz), 2.80-2.92 (4H, m), 3.55 (3H, s), 4.22 (2H, t, **J *=*** 6.6 Hz), 4.94 (2H, s), 5.05 (2H, s), 6.56 (1H, d, J = 8.7 Hz), 7.41 (1H, d, J = 8.7 Hz), 7.76 (2H, s), 7.80 (1H, s), 8.09 (2H, s)

A solution of 3-{1-[({3-[(cyclopentylmethyl)(ethyl)amino]-6-methoxypyridin-2-yl}methyl){5-[2-(methylthio)ethoxy]pyrimidin-2-yl}amino]methyl}-5-(trifluoromethyl)henzonitrile (1.37 g, 2.23 mmol) in acetonitrile (30 mL) was added with molybdenum dioxide dichloride (67 mg, 0.335 mmol) and 30% aqueous hydrogen peroxide (1.05 g, 9.28 mmol), and the mixture was stirred at room temperature for 21 hours. The reaction mixture was added with saturated aqueous sodium sulfite, and extracted with ethyl acetate, and the organic layer was washed with saturated brine, dried over anhydrous sodium sulfate, and concentrated under reduced pressure. The resulting residue was purified by using silica gel column chromatography (hexane:acetone = 4:1) to obtain 3-{1-[({3-[(cyclopentylmethyl)(ethyl)amino]-6-methoxypyridin-2-yl}methyl){5-[2-(methylsulfonyl)ethoxy]pyrimidin-2-yl}amino]methyl}-5-(trifluoromethyl)benzonitrile (1.17 g, 81%) as yellow oil. This yellow oil was crystallized from methanol to obtain colorless crystalline powder.
Melting point: 97.2-98.2°C
IR (ATR) cm⁻¹: 2949, 2867, 1607, 1552, 1500, 1473
¹H-NMR (400 MHz, CDCl₃) δ: 0.95 (3H, t, J = 7.1 Hz), 1.06-1.20 (2H, m), 1.37-1.67 (6H, m), 1.87 (1H, m), 2.77 (2H, d, J = 7.6 Hz), 2.88 (2H, q, J = 7.1 Hz), 3.07 (3H, s), 3.43 (2H, t, J = 5.3 Hz), 3.56 (3H, s), 4.40 (2H, t, J = 5.3 Hz), 4.95 (2H, s), 5.05 (2H, s), 6.58 (1H, d, J= 8.8 Hz), 7.42 (1H, d, J = 8.8 Hz), 7.76 (2H, s), 7.79 (1H, s), 8.10 (2H, s)

### Comparative Example 1

The demethylation was performed in the same manner as that of Example 2 with the conditions shown in Table 1. The results are shown in Table 1. As can be understood from the results shown in Table 1, the reaction of Example 3 gave the highest yield. On the other hand, under the other reaction conditions, the yield was markedly reduced, or no target substance was obtained, because the reaction did not advance, or a complicated product was obtained.

**[Table 1]**

| Example | Reagent | Solvent | Equivalent | Reaction conditions | Result |
|---|---|---|---|---|---|
| 1 | AlCl₃ | (CH₂Cl)₂ | 1.5 | 70°C, 7h. | Reaction did not occur |
| 2 | AlCl₃ | (CH₂Cl)₂ | 2.0 | 70°C, 7 h | Reaction was not completed |
| 3 | AlCl₃ | (CH₂Cl)₂ | 3.0 | 70°C, 2 h | 100 % |
| 4 | BBr₃ | (CH₂Cl)₂ | 3.0 | 80°C, 0.5 h | 85% |
| 5 | 57% HI | Water | Excess | 90°, 3 h | Decomposition |
| 6 | 30% HBr | Acetic acid | Excess | 90°C, 7 h | Decomposition |
| 7 | (CH₃)ₐSiI | CHCl₃ | 1.5 | 25°C, 15 h | Reaction did not occur |
| 8 | LiI | Lutidine | 2.0 | 150°C, 13 h | 46% |
| 9 | LiC1 | DMF | 3.0 | 150°C, 13 h | Reaction did not occur |
| 10 | NaCN | DMSO | 5.0 | 180°C, 5 h | Decomposition |
| 11 | SiCl₄ NaI | Toluene | 1.1 | 90°C, 15 h | Reaction did not occur |

### Comparative Example 2

The etherification was performer in the same manner as that of Example 3 with the conditions shown in Table 2. The results are shown in Table 2. As can be understood from the results shown in Table 2, the reaction conditions of Example 19 gave a high yield with a short time. On the other hand, under the other reaction conditions, the yield was markedly reduced or the reaction did not advance, or complex mixture was obtained.

**[Table 2]**

| Example | Base (eq.) | Reagent (eq.) | Solvent | Reaction conditions | Result |
|---|---|---|---|---|---|
| 1 | K₂CO₃ (3.0) | 1.5 | NMP | 120°C, 4 days | Reaction was not completed |
| 2 | NaH (1.2) | 1.5 | DMF | 100°C, 2 days | Reaction was not completed |
| 3 | CsOH H₂O(10) | 3.0 | (CH₂Cl)₂ | 90°C, 2 days | By-product |
| 4 | NaOH (5.0) | 3.0 | MeOH | 70°C, 3 h | 55% |
| 5 | NaOH (5.0) | 3.0 | t-BuOH | 90°C, 3 h | 68% |
| 6 | LiOH (5.0) | 3.0 | t-BuOH | 90°C, 44 h | 64% |
| 7 | KOH (5.0) | 3.0 | t-BuOH | 90°C, 24 h | 40% |
| 8 | CsOH (5.0) | 3.0 | t-BuOH | 90°C, 50 h | 20% |
| 9 | tBuOK (5.0) | 3.0 | t-BuOH | 90°C, 50 h | 4.7% |
| 10 | NaOH (3.0) | 3.0 | t-BuOH H₂O 10 : 1 | 90°C, 3 h | 69% |
| 11 | NaOH (5.0) | 3.0 | i-PrOH | 90°C, 3 h | 42% |
| 12 | NaOH (5.0) | 3.0 | DMF | 90°C, 1,5 h | 51% |
| 13 | NaOH (2.0) | 1.5 | t-BuOH | 90°C, 11 h | 82% |
| 14 | NaOH (2.0) | 1.5 | i-PrOH | 90°C, 9.5 h | 50% |
| 15 | NaOH (2.0) | 1.5 | CH₃CN | 90°C, 7 h | 66% |
| 16 | NaOH (2.0) | 1.5 | DMF | 90°C, 3 h | 65% |
| 17 | NaOH (1.2) | 1.2 | t-BuOH | 90°C, 11 h | 80% |
| 18 | NaOH (1.0) | 1.0 | t-BuOH | 90°C, 12 h | 79% |
| 19 | NaOH (1.0) | 1.0 | i-PrOH | 90°C, 3 h | 80% |
| 20 | NaOH (1.0) | 1.0 | DMF | 90°C, 12 h | 46% |

### Industrial Applicability

According to the method of the present invention, 5-[2-(methylthio)ethoxy]pyrimidin-2-amine, which is useful as a raw material for manufacture of medicaments, agricultural chemicals, and industrial products, can be efficiently prepared with convenient reaction conditions.

## Claims

1. A method for preparing 5-[2-(methylt.hio)ethoxy]pyrimidin-2-amine represented by the following formula (5): 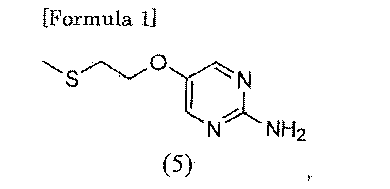 which comprises the following steps:
a) the step of reacting a compound represented by the following formula (3): 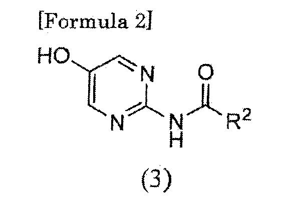 [in the formula, R² represents a lower alkyl group] with a 2-haloethyl methyl sulfide in an alcoholic solvent in the presence of an alkaline metal hydroxide to prepare a compound represented by the following formula (4): 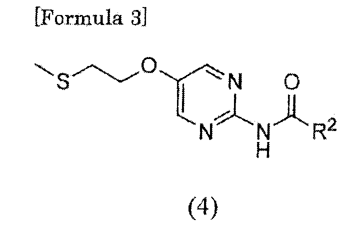 [in the formula, R² represents the same group as defined above], and
b) the step of treating the compound represented by the formula (4) with a base in a solvent to prepare a compound represented by the formula (5).

2. The method according to claim 1, wherein the 2-haloethyl methyl sulfide is 2-chloroethyl methyl sulfide.

3. The method according to claim 1 or 2, wherein R² is n-pentyl group.

4. The method according to any one of claims 1 to 3, wherein the alkaline metal hydroxide is sodium hydroxide.

5. The method according to any one of claims 1 to 4, wherein the alcoholic solvent is methanol, ethanol, isopropanol, or t-butanol.

6. The method according to any one of claims 1 to 5, wherein the step b) is performed without isolating the compound represented by the formula (4) obtained in the step a).

7. The method according to any one of claims 1 to 6, which further comprises the step of treating a compound represented by the following formula (2): 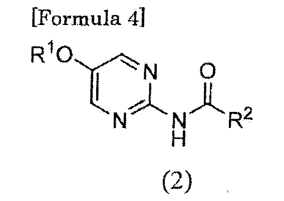 [in the formula, H¹ and R² independently represent a lower alkyl group] with aluminum chloride in a halogenated hydrocarbon solvent to prepare a compound represented by the formula (3).

8. The method according to claim 7, wherein R¹ is methyl group.

9. The method according to claim 7 or 8, wherein the halogenated hydrocarbon solvent is 1,2-dichloroethane.

10. The method according to any one of claims 1 to 9, which further comprises the step of reacting a compound represented by the following formula (1): 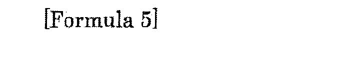 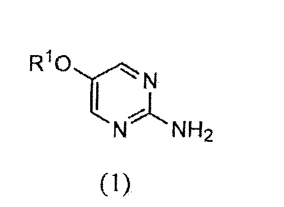 [in the formula, R¹ represents a lower alkyl group] with an alkanoic acid halide in a solvent in the presence of an organic base to prepare a compound represented by the formula (2).

11. The method according to claim 10, wherein the organic base is pyridine.

12. The method according to claim 10 or 11, wherein the alkanoic acid halide is cyclohexanoyl chloride.

13. The method according to any one of claims 10 to 12, wherein the solvent is a halogenated hydrocarbon solvent.

14. A method for preparing 3-{1-[({3-[(cyclopentylmethyl)(ethyl)amino]-6-methoxypyridin-2-yl}methyl){5-[2-(methylsulfonyl)ethoxy]pyrimidin-2-yl}amino]methyl}-5-(trifluoromethyl)benzonitrile represented by the following formula (A): 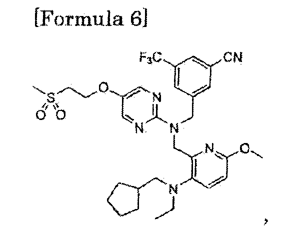 which comprises the steps described in any one of claims I to 13.
